Europäisches Patentamt

European Patent Office

Office européen des brevets

(11) Veröffentlichungsnummer: **0 496 090 A2**

# EUROPÄISCHE PATENTANMELDUNG

(21) Anmeldenummer: **91121912.9**

(22) Anmeldetag: **20.12.91**

(51) Int. Cl.5: **C07C 49/10**, C07C 45/79, C07C 45/81, C07C 43/13, C07C 41/36, B01D 61/00

(30) Priorität: **15.01.91 DE 4100984**

(43) Veröffentlichungstag der Anmeldung: **29.07.92 Patentblatt 92/31**

(84) Benannte Vertragsstaaten: **AT BE CH DE ES FR GB IT LI NL SE**

(71) Anmelder: **BASF Aktiengesellschaft Carl-Bosch-Strasse 38 W-6700 Ludwigshafen(DE)**

(72) Erfinder: **Krug, Joseph, Dr. Franz-von-Sickingen-Strasse 9 W-6700 Ludwigshafen(DE)**
Erfinder: **Baumgart, Hubert, Dr. Lange Kule 16 W-4400 Muenster(DE)**

(54) **Verfahren zur Trennung eines Gemisches aus Wasser, Methylethylketon und Ethylenglykol-mono-n-butylether in Wasser und Methylethylketon mit Ethylenglykol-mono-n-butylether.**

(57) Die Erfindung betrifft ein Verfahren zur Trennung eines Gemisches aus Wasser, Methylethylketon und Ethylenglykol-mono-n-butylether in Wasser und Methylethylketon mit Ethylenglykol-mono-n-butylether, wobei die Trennung mittels Pervaporation durchgeführt wird.

## FIG.2

EP 0 496 090 A2

Die Erfindung betrifft ein Verfahren zur Trennung eines Gemisches aus jeweils Wasser,
Methylethylketon oder
Methylethylketon mit Ethylenglykol-mono-n-butylether- oder
Aceton oder
Methylpropylketon oder
Methylisobutylketon oder
Diethylketon oder
Diisobutylketon oder
Ethylamylketon oder
Cyclohexanen oder
Isophoren
in jeweils Wasser und die obigen Produkte.

Methylethylketon ist ein häufig verwendetes Lösungsmittel in der chemischen Industrie, beispielsweise als Lösungsmittel für chemische Reaktionen und als Lösungsmittel für Ausgangsstoffe bestimmter Verbindungen. Nach dem Einsatz fällt dabei das Methylethylketon mit dem Ethylenglykol-mono-n-butylether oft wasserhaltig an.

Da sich das Gemisch beispielsweise durch einfache Destillation/Rektifikation nur bis zum Azeotrop auftrennen läßt - das Gemisch besitzt neben einer Mischungslücke ein zusätzliches homogenes Azeotrop außerhalb der Mischungslücke - und die üblichen Methoden zur Stofftrennung von Azeotropen wie beispielsweise Druckwechselverfahren, Schleppmitteldestillation etc. nicht zum Erfolg führen, fallen bei der Rückführung des Methylethylketons mit dem Ethylenglykol-mono-n-butylether zwangsläufig hohe Verluste an. Die organische Phase aus der Mischungslücke mit annähernd azeotroper Zusammensetzung mußte bisher aufwendig entsorgt werden, beispielsweise durch Verbrennung.

Der Erfindung liegt daher die Aufgabe zugrunde, das Gemisch aus Wasser, Methylethylketon und Ethylenglykolmono-n-butylether ohne große Verluste zu entwässern.

Die Aufgabe wird erfindungsgemäß dadurch gelöst, daß die Trennung mittels Pervaporation durchgeführt wird. Hierbei wird das Gemisch flüssig oder gasförmig über eine hydrophile Membrane geführt.

Das Verfahren kann technisch sowohl in Batch- als auch in kontinuierlicher Fahrweise betrieben werden. Beide Verfahrensmöglichkeiten werden nachstehend detailliert beschrieben.

Beispiel 1

Batchfahrweise, Entwässerung von Methylethylketon mit Ethylenglykol-mono-n-butylether durch Pervaporation.

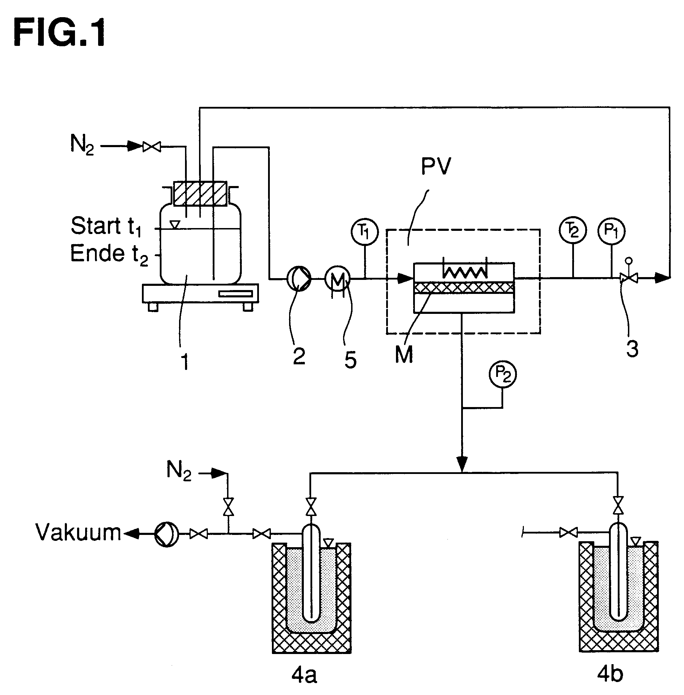

Das diskontinuierlich durchgeführte Verfahren ist in Figur 1 dargestellt. Dabei wird ein Gemisch aus 88 Gew.-% Methylethylketon einschließlich ca. 1 Gew.-% Ethylenglykol-mono-n-butylether und 12 Gew.-% Wasser mit 800 g im Behälter 1 bei Raumtemperatur vorgelegt. Diese Vorlaufmenge wird über Pumpe 2 und die Testzelle PV mit der Membranfläche M von 100 cm$^2$ ca. 5 mal in der Stunde im Kreis gefahren. Am Druckhalteventil 3 wird ein Überdruck von 2,6 bar eingestellt. Auf der Permeatseite liegt ein absoluter Druck von 30 mbar an. Das Permeat wird in den Kühlfallen 4a oder 4b bei ca. -70°C ausgefroren. Die Analyse des Permeats und des Behälterinhalts erfolgt in den ersten 6 Stunden stündlich, danach im Durchschnitt alle 7 Stunden. Nach der An- Einfahrweise wird das Feed vom Thermostaten 5 innerhalb einer Stunde von Raumtemperatur auf 95°C erwärmt und konstant gehalten (Zeitpunkt t1). Der Temperaturunterschied zwischen der Feedtemperatur T1 (PV-Ein) und der Retentattemperatur T2 (PV-Aus) beträgt dann zum Zeitpunkt t1 ca. 15°C zum Zeitpunkt t2 ca. 3°C und mit Mittel ca. 6°C. Nach 52 Stunden (Zeitpunkt t2) ergibt sich eine Endproduktmenge von 702,4 g mit 99,95 Gew.-% Methylethylketon einschließlich Ethylenglykol-mono-n-butylether und ca. 500 Gew.-ppm Wasser. Durch die hydrophile Membran sind 97,6 g mit 98 Gew.-% Wasser und 2 Gew.-% Methylethylketon permeiert. Der Fluß hat dabei von 2,5 kg/m$^2$h auf ca. 0,01 kg/m$^2$h abgenommen.

Beispiel 2

Kontinuierliche Fahrweise, Entwässerung von Methylethylketon mit Ethylenglykol-mono-n-butylether durch Pervaporation.

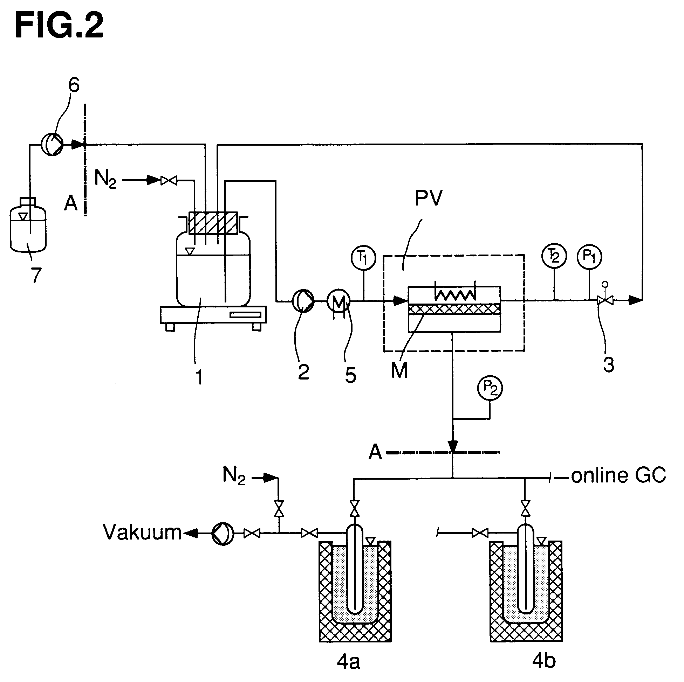

Das kontinuierlich durchgeführte Verfahren ist in der Figur 2 dargestellt. Dabei wird ein Gemisch aus 94,7 Gew.-% Methylethylketon einschließlich ca. 1 Gew.-% Ethylenglykol-mono-n-butylether und 5,3 Gew.-% Wasser mit ca. 6 kg im Behälter 1 bei Raumtemperatur vorgelegt. Die Pumpe 2 fördert ca. 3,5 kg/h im Kreis. Die Membranfläche M der Pervaporation beträgt 100 cm$^2$. Am Druckhalteventil 3 wird ein Überdruck von 2,6 bar eingestellt. Auf der Permeatseite liegt ein absoluter Druck von 30 mbar an. Das Permeat wird entweder in den Kühlfallen 4a oder 4b bei ca. -70°C ausgefroren oder im GC-Onlinebetrieb analysiert. Nach der Anfahrweise wird das Feed vom Thermostaten 5 innerhalb einer Stunde von Raumtemperatur auf ca. 80°C erwärmt und konstant gehalten. Der Temperaturunterschied zwischen der Feedtemperatur T1 (PV-Ein) und der Retentattemperatur T2 (PV-Aus) beträgt dann ca. 1°C. Über eine Dosierpumpe 6 wird aus der Vorlage 7 dem Behälter B1 ungefähr soviel Gemisch zugeführt, wie Permeat bei der Pervaporation anfällt (Schnitt A-A). Das Permeat besteht aus 99,6 Gew.-% Wasser und ca. 0,4 Gew.-% Methylethylketon. Der Fluß beträgt ca. 0,7 kg/m$^2$h. Diese stationäre Betriebsweise mit konstanter Feedzusammensetzung wurde über einen Zeitraum von ca. 77 Stunden gefahren.

Die mit der Erfindung erzielten Vorteile bestehen darin, daß durch die Aufarbeitung des Azeotrops das Lösungsmittel Methylethylketon zurückgewonnen und damit in den Prozeß zurückgeführt werden kann. Außerdem entfallen zusätzlich die Kosten für die Verbrennung der organischen Phase aus der Mischungslücke mit annähernd azeotroper Zusammensetzung.

**Patentansprüche**

1. Verfahren zur Trennung eines Gemisches aus
   jeweils Wasser,
   Methylethylketon oder
   Methylethylketon mit Ethylenglykol-mono-n-butylether- oder
   Aceton oder
   Methylpropylketon oder
   Methylisobutylketon oder
   Diethylketon oder
   Diisobutylketon oder
   Ethylamylketon oder
   Cyclohexanon oder
   Isophoren
   in jeweils Wasser und die obigen Produkte, dadurch gekennzeichnet, daß die Trennung mittels Pervaporation durchgeführt wird.

2. Verfahren gemäß Anspruch 1, dadurch gekennzeichnet, daß die Trennung mittels einer hydrophilen Membrane durchgeführt wird.

# FIG.1

# FIG.2